# EUROPEAN PATENT APPLICATION

(11) **EP 1 563 845 A1**
(43) Date of publication of application: **17.08.2005**
(21) Application number: 03754059.8
(22) Date of filing: 09.10.2003
(51) Int. Cl.: A61K 45/00, A61K 31/5575, A61P 11/02, A61P 11/06, A61P 17/00, A61P 27/02, A61P 29/00, A61P 37/08, A61P 43/00

(54) **REMEDIES FOR ALLERGIC DISEASES**

(30) Priority: 10.10.2002 JP 2002297900
(71) Applicant: ONO PHARMACEUTICAL CO., LTD., Osaka-shi, Osaka 541-8526 (JP); Narumiya, Shuh, Kyoto 606-0007 (JP)
(72) Inventor: Narumiya, Shuh, Kyoto-shi, Kyoto 606-0007 (JP)
(74) Representative: McCluskie, Gail Wilson
(86) International application number: PCT/JP2003/012980
(87) International publication number: WO 2004/032964

(57) **Abstract**

The present invention relates to a prophylactic and/or therapeutic agent for an allergic disease which contains a compound having an agonistic activity to EP3 receptor that is one of the prostaglandin E2 receptor subtypes. More specifically, a compound having an agonistic activity to EP3 receptor is effective in therapy of an allergic respiratory disease such as bronchial asthma, pediatric asthma, allergic asthma, or atopic asthma, and a selective agonistic compound can be expected to induce a more remarkable therapeutic effect.

## Description

### Technical Field

The present invention relates to a prophylactic and/or therapeutic agent for an allergic disease. More specifically, the present invention relates to a prophylactic and/or therapeutic agent for an allergic disease which contains a compound having an agonistic activity to EP3 receptor that is one of the prostaglandin E2 receptor subtypes.

### Background Art

It is estimated that 20% or more the population has abnormal allergy reactions that are maj or causes of asthma, hay fever, rhinitis, or dermatitis. The number of population having such reactions is recognized as a social problem (Lancet, 351, 1225 (1998), Science, 296, 490 (2002), Nature, 402, B2 (1999)). Those allergy reactions are caused by the process of acting a complex formed by binding an allergen such as a house dust or pollen to IgE on an IgE receptor on a mast cell. When the activated mast cell liberates various chemical mediators including histamine to cause development of allergy reactions or inflammatory condition, large amounts of lipid mediators such as prostaglandin (hereinafter, abbreviated as PG) and leukotriene (hereinafter, abbreviated as LT) are also produced (Clin Allergy Immunol, 16, 223, (2002)). Of those lipid mediators, PGD2, LTB4, LTC4, and LTD4 are known to cause migration of inflammatory cells typified by eosinophils to promote allergic symptoms.

On the other hand, prostaglandin E2 (hereinafter, abbreviated as PGE2) produced in a large amount is known to have various physiological activities such as a cyto-protective activity, suppressive effect on gastric acid secretion, uterine contractile activity, promoting effect on digestive peristalsis, pain-inducing effect, awaking effect, and hypotensive activity, but actions on allergic reactions have been unclear.

The recent studies have found that the PGE2 receptor is divided into some subtypes which possess roles different from each other. At present, four main receptor subtypes are known and they are called EP1, EP2, EP3, andEP4, respectively (J.B.C, 268, 35, 26767- (1993), Mol. Pharamacol, 46, 213-(1994), B.B.R.C, 198, 99- (1994), J.B.C, 269, 11873- (1994), J. Lipid Mediators Cell Signalling, 12, 379-391 (1995)). When PGE2 binds to each of those receptors, various physiological actions are exerted. However, when PGE2 binds to plural subtypes of the receptors, the actions sometimes contradict each other, so that the unique action of each subtype is possibly difficult to be detected. Therefore, the inventors of the present invention created EP3 receptor-deficient mice and synthesized a compound having an agonistic activity to EP3 receptor, and those have been used for study on a physiological role of an EP3 receptor.

The EP3 receptor is known to be involved in signaling of the peripheral nerve (Biochem. J., 340, 365- (1999)), control of an exoergic reaction in the nerve center (Nature, 395, 281- (1998)), memory formation by expression in the brain neuron (J. Comp. Neurol. , 421, 543- (2000)), angiogenesis (The Japanese Journal of Pharmacology, 117(4), 283- (2001)), primitive urine reabsorption by expressing in the renal tubular (Kidney Int Suppl, 55, S183-(1996), uterine contractile activity, ACTH production, and platelet agglutination (Circulation, 104, 1176- (2001)) and to be expressed also in the vascular smooth muscle, heart, and digestive tract. However, the receptor has not been known to be specifically involved in allergy crisis.

The inventors of the present invention have studied on involvement of EP3 receptor in allergy reactions using the method already reported in a literature (Science, 287, 2013- (2000)). Specifically, the asthma condition was induced by inhalation of ovalbumin (hereinafter, abbreviated as OVA) in an EP3 receptor-deficient mouse, and the lung condition was analyzed. As a result, the amounts of infiltrated cells in the lung tissue/alveoli and liberated Th2 cytokine in the EP3-deficient mouse have been found to significantly increase compared to a wild-type mouse. Moreover, the amounts of histamine and LT released from the extracted lung of a sensitized wild-type mouse by in vitro antigen stimulation have been found to increase by indomethacin which is a PGH-producing enzyme (cyclooxygenase) inhibitor and to be suppressed by an agonist for the EP3 receptor. Although those results have been disclosed, the experiment using the EP3 receptor-deficient mouse has found that the EP3 receptor-deficient mouse showed a promoted allergic asthma symptom compared to a wild-type mouse, while the wild-type mouse showed a significant asthma symptom. The results showed that PGE2 was produced by antigen stimulation in the animals, and it acts on the EP3 receptor in the wild-type mouse, resulting in alleviation of an asthma attack. However, such an experiment has never revealed that EP3 receptor stimulation by the agonist for EP3 receptor surpassed such feedback inhibition and induced suppression of an asthma allergy symptom in the wild-type mouse. In addition, the suppression of the amount of histamine released from the sensitized lung by the agonist for the EP3 receptor causes only improvement of the portion enhanced by indomethacin. Therefore, the effect on a type of asthma which was considered to be caused by ingestion of a cyclooxygenase inhibitor (so-called aspirin asthma) was expected, but the effect on various allergic diseases caused by antigens present in the life environments was not suggested.

### Disclosure of the Invention

The inventors of the present invention have paid notice to a compound having an agonistic activity to EP3 receptor as a novel target in therapy of allergy. An object of the present invention is to provide a prophylactic and/or therapeutic agent for an allergic disease, which is characterized by selectively acting via EP3 receptor.

The inventors of the present invention have studied whether administration of the compound having an agonistic activity to EP3 receptor is effective in therapy of an allergic disease using allergic disease animal models. As a result, they have found out that the compound having an agonistic activity to EP3 receptor has an effect on therapy and/or prophylaxis of an allergic disease, thereby completing the present invention.

That is, the present invention relates to use of a compound having an agonistic activity to EP3 receptor for producing:
(1) a prophylactic and/or therapeutic agent for an allergic disease which contains a compound having an agonistic activity to EP3 receptor;
(2) the prophylactic and/or therapeutic agent for an allergic disease according to the item (1) above, in which the allergic disease is an allergic respiratory disease, allergic nasal disease, allergic skin disease, or allergic ocular disease;
(3) the prophylactic and/or therapeutic agent for an allergic disease according to the item (2) above, in which the allergic respiratory disease is bronchial asthma, pediatric asthma, allergic asthma, or atopic asthma, the allergic nasal disease is allergic rhinitis, vernal catarrh, hay fever, or chronic allergic rhinitis, the allergic skin disease is atopic dermatitis, or the allergic ocular disease is seasonal allergic conjunctivitis, hay fever, or chronic allergic conjunctivitis;
(4) the prophylactic and/or therapeutic agent for an allergic disease according to the item (2) above, in which the allergic respiratory disease is bronchial asthma, pediatric asthma, allergic asthma, or atopic asthma;
(5) the prophylactic and/or therapeutic agent for an allergic disease according to the item (1) above, in which the compound having an agonistic activity to EP3 receptor is a compound represented by the formula (I): 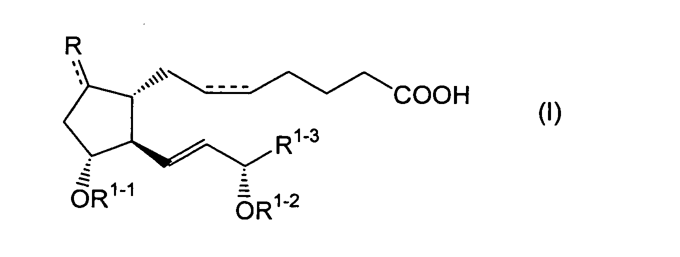 [wherein R represents an oxo group or a halogen atom, R¹⁻¹ and R¹⁻² each independently represent a C1-4 alkyl group, and R¹⁻³ represents a C1-10 alkyl group, a C2-10 alkenylene group, or a C2-10 alkynylene group substituted by a C1-10 alkyl group, a C2-10 alkenylene group, a C2-10 alkynylene group, a phenyl group, a phenoxy group, a C3-7 cycloalkyl group, or a C3-7 cycloalkyloxy group. The phenyl and cycloalkyl groups may be substituted by 1-3 of C1-4 alkyl groups, C1-4 alkoxy groups, halogen atoms, trihalomethyl groups, or nitro groups] or a salt or a solvate thereof;
(6) the prophylactic and/or therapeutic agent for an allergic disease according to the item (5) above, which is 11α,15α-dimethoxy-9-oxoprosta-5Z,13E-dienoic acid or a salt or solvate thereof;
(7) the prophylactic and/or therapeutic agent for an allergic disease according to the item (1) above, in which the compound having an agonistic activity to EP3 receptor is misoprostol or sulprostone;
(8) a pharmaceutical composition including the compound having an agonistic activity to EP3 receptor described in the item (1) above in combination with one or more drugs selected from anti-asthma drugs, inhaled steroid drugs, inhaled β2 stimulants, methylxanthine asthma drugs, anti-allergy drugs, histamine H1-antagonists, antiinflammatory drugs, anti-choline drugs, thromboxane antagonists, leukotriene antagonists, LTD4 antagonists, PAF antagonists, phosphodiesterase inhibitors, β2 agonist, steroid drugs, mediator release-suppressing drugs, eosinophil chemotactic-suppressing drugs, disodium cromoglycate, macrolide antibiotics, immune-suppressing agent, and hyposensitization therapy agents;
(9) a method of preventing and/or treating an allergic disease, which is characterized by including administering an effective amount of a compound having an agonistic activity to EP3 receptor to a mammal; and
(10) use of a compound having an agonistic activity to EP3 receptor for producing a prophylactic and/or therapeutic agent for an allergic disease.

The inventors of the present invention have analyzed an EP3 (one of the PGE2 receptor subtypes) gene-deficient mouse and have already elucidated that EP3 is involved in crisis of an allergic reaction from the results of significant increase in the amount of infiltrated eosinophilics in a bronchoalveolar lavage fluid (hereinafter, abbreviated as BALF), and more significant increase in the number of infiltrated cells into a lung tissue and in the amount of Th2 cytokine in the mouse. Furthermore, they have found out that the amount of a chemical mediator that is released by in vitro antigenic stimulation of the lung of a sensitized wild-type mouse is increased by indomethacin which is a PGH-producing enzyme (cyclooxygenase) inhibitor and is suppressed by an agonist for EP3 receptor. However, the suppression leads to only improvement of the portion enhanced by indomethacin. Therefore, an effect was expected only on so-called aspirin asthma, which was considered to be caused by ingestion of a cyclooxygenase inhibitor, but it was considered that an effect was not expected on other many allergic diseases caused by antigens.

The inventors of the present invention have further studied earnestly on action of a compound having an agonistic activity to EP3 receptor on an allergic reaction caused by antigen sensitization using an antigen-sensitized asthma model mouse. Specifically, an asthma reaction was induced using a mouse that had previously been sensitized with an antigen of OVA. Subsequently, they have studied on the change in the number of inflammatory cells in BALF serving as an indicator of an allergic reaction. As a result, as shown in Example 1, it was found that the eosinophil and neutrophil numbers in BALF decreased significantly, and the allergic reaction was suppressed by administration of a compound having an agonistic activity to EP3 receptor. That is, the pathogenic condition was cured by administration of the compound having an agonistic activity to EP3 receptor to the asthma model mouse. This showed that the compound having an agonistic activity to EP3 receptor is useful in therapy and/or prophylaxis not only of aspirin asthma but also of many allergic diseases caused by exposure to antigens.

The compound having an agonistic activity to EP3 receptor to be used in the present invention may be any one of the compounds each having an agonistic activity to EP3 receptor, and examples thereof include a compound selectively having an agonistic activity to EP3 and a compound specifically having an agonistic activity to EP3.

The compound selectively having an agonistic activity to EP3 may have any agonistic activities to a prostaglandin receptor other than EP3. Preferably, the compound contains a substance in which the strongest activity of those agonistic activities is 1/10 or less or 1/100 or less, preferably 1/1, 000 or less than the agonistic activity to EP3. Meanwhile, the compound specifically having an agonistic activity to EP3 contains a substance in which any agonistic activities to a prostaglandin receptor other than EP3 are 1/10 or less or 1/100 or less, preferably 1/1,000 or less, more preferably 1/10,000 or less than the agonistic activity to EP3.

In the formula of the compound according to the present invention, as may be easily understood by those skilled in the art, unless otherwise specified, the symbol 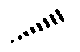 indicates that the substituents attached thereto is in front of the sheet (i.e., α-configuration), the symbol 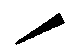 indicates that the substituents attached thereto is behind the sheet (i.e., β-configuration), the symbol 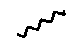 indicates α-configuration, β-configuration, or a mixture thereof, the symbol 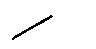 indicates a mixture of α-configuration and β-configuration, the symbol 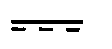 indicates a single bond or a double bond, the symbol 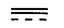 indicates a double bond or a triple bond, and the symbol 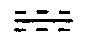 indicates a single bond, a double bond, or a triple bond.

The compounds to be used in the present invention encompass all isomers thereof unless otherwise specified. For example, an alkyl group, an alkenyl group, an alkynyl group, an alkoxy group, an alkylthio group, an alkylene group, an alkenylene group, and an alkynylene group include linear and branched-chain isomers. Furthermore, the present invention includes isomers in a double bond, ring, or condensed ring (E, Z, cis, and trans forms), isomers due to presence of an asymmetric carbon or the like (R or S form, α or β configuration, enantiomer, and diastereomer), optically-active substances having an optical activity (D, L, d, and 1 forms), polar forms obtained by chromatograph separation (highly polar form and lowly polar form), equilibrium compounds, rotational isomers, mixtures including those compounds at an optional rate, and racemic mixtures.

Examples of the compound having an agonistic activity to EP3 in the present invention include compounds described in the pamphlet of WO 98/34916. Of those, examples of preferable compounds include a compound represented by the formula (I): 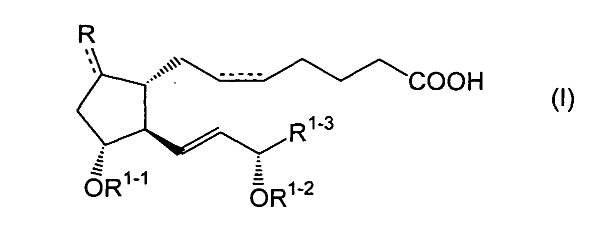 [wherein R represents an oxo group or a halogen atom, R¹⁻¹ and R¹⁻² each independently represent a C1-4 alkyl group, and R¹⁻³ represents a C1-10 alkyl group, a C2-10 alkenylene group, or a C2-10 alkynylene group substituted by a C1-10 alkyl group, a C2-10 alkenylene group, a C2-10 alkynylene group, a phenyl group, a phenoxy group, a C3-7 cycloalkyl group, or a C3-7 cycloalkyloxy group. The phenyl and cycloalkyl groups may be substituted by 1-3 of C1-4 alkyl groups, C1-4 alkoxy groups, halogen atoms, or trihalomethyl groups, nitro groups] or a salt or a solvate thereof.

In the formula (I), the term "C1-4 alkyl group" means methyl, ethyl, propyl, butyl, and branched isomer groups thereof; the term "C1-4 alkoxy group" means methoxy, ethoxy, propoxy, butoxy, and branched isomer groups thereof; the term "C1-10 alkyl group" means methyl, ethyl, propyl, butyl, pentyl, hexyl, heptyl, octyl, nonyl, decyl, and branched isomer groups thereof; the term "C2-10 alkenyl group" means vinyl, propenyl, butenyl, pentenyl, hexenyl, heptenyl, octenyl, nonenyl, decenyl, and branched isomer groups thereof; the term "C2-10 alkynyl group" means ethynyl, propynyl, pentynyl, hexynyl, heptynyl, octynyl, nonynyl, decynyl, and branched isomer groups thereof; and the term "C3-7 cycloalkyl group" means cyclopropyl, cyclobutyl, cyclopentyl, cyclohexyl, and cycloheptyl groups. In the formula (I), the term "halogen atom" means fluorine, chlorine, bromine, and iodine atoms.

Of compounds represented by the formula (I), examples of more preferable compounds include 11α,15α-dimethoxy-9-oxoprosta-5Z,13E-dienoic acid (see the pamphlet of WO 98/34916) or a salt or a solvate thereof.

Furthermore, examples of the compound having an agonistic activity to EP3 of the present invention include the compounds described in the specification of US 5624959 or US 5723493. Of those, examples of preferable compounds include a compound represented by the formula (II): 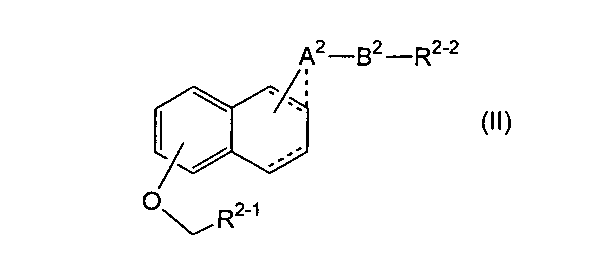 [wherein R²⁻¹ represents a -COOR²⁻⁴ group (wherein R²⁻⁴ represents a hydrogen atom or C1-4 alkyl group), -CONR²⁻⁵R²⁻⁶ group (wherein R²⁻⁵ and R²⁻⁶ each independently represent a hydrogen atom, a C1-4 alkyl group, or a C1-4 alkyl group in which one hydroxyl group is substituted), or -CH₂OH, 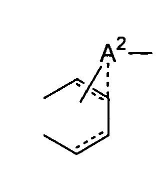 represents 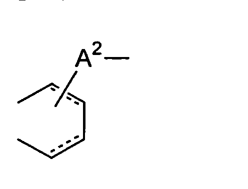 (wherein A² represents a single bond or a C1-4 alkylene group), or 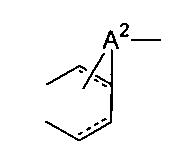 or 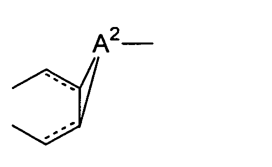 (wherein A² represents a group represented by: 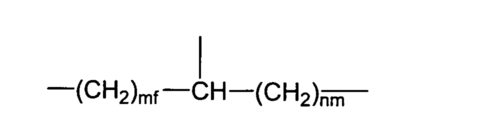 (wherein mf and nm each are 0 or an integer of 1 to 4, with the proviso that mf + nm is an integer of 2-4)), B² represents -NR²⁻³SO₂- or -SO₂NR²⁻³- (wherein R²⁻³ represents a hydrogen atom, a C1-4 alkyl group, or -CH₂COOR²⁻⁷- (wherein R²⁻⁷ represents a hydrogen atom or R^{2-4a} (wherein R^{2-4a} represents a C1-4 alkyl group)), R²⁻² represents (1) a C1-6 alkyl group, a C2-6 alkenyl group, or a C2-6 alkynyl group, (2) a C1-6 alkyl group, a C2-6 alkenyl group, or a C2-6 alkynyl group substituted by a phenyl group substituted by 1-3 of substituents selected from 1-3 of phenyl groups, C4-7 cycloalkyl groups, or C1-4 alkyl groups, C1-4 alkoxy groups, or halogen atoms, or (3) a naphthyl group, and in the following formula: 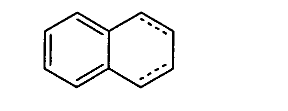 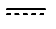 represents a single bond or a double bond] or a salt or a solvate thereof.

Moreover, examples of the compoundhaving an agonistic activity to EP3 of the present invention include the compounds described in the specification of US 5753700 or US 6013673. Of those, examples of preferable compounds include a compound represented by the formula (III): 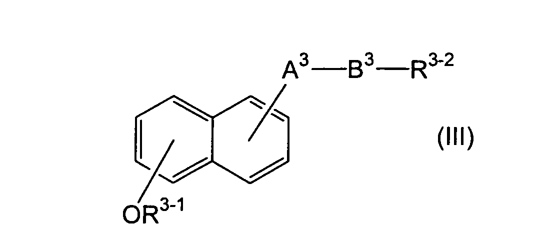 [wherein R³⁻¹ represents a hydrogen atom, a C1-4 alkyl group, a group represented by the formula (C1-4 alkylene)-COOR³⁻¹⁰ group (wherein R³⁻¹⁰ represents a hydrogen atom or a C1-4 alkyl group), a (C1-4 alkylene)-OH group, group represented by the formula (C1-4 alkylene) -CONR³⁻⁴R³⁻⁵ (wherein R³⁻⁴ and R³⁻⁵ each independently represent a hydrogen atom or a C1-4 alkyl group), group represented by the formula (C1-4 alkylene) -CONR³⁻⁶- (C1-4 alkylene)-OH (wherein R³⁻⁶ represents a hydrogen atom or a C1-4 alkyl group), group represented by the formula (C1-4 alkylene)-NR³⁻⁴R³⁻⁵ (wherein R³⁻⁴ and R³⁻⁵ are as defined above), a (C1-4 alkylene)-CN group, or a (C1-4 alkylene)-tetrazolyl group, A³ represents a single bond, a C1-6 alkylene group, a C2-6 alkenylene group, a -S-(C1-6 alkylene) group, or a -O-(C1-6 alkylene) group, B³ represents a group represented by the formula NR³⁻³CO or CONR³⁻³ (wherein R³⁻³ represents a hydrogen atom or a C1-4 alkyl group), R³⁻² represents (1) a C1-6 alkyl group, (2) a C2-6 alkenyl group, (3) a C1-6 alkyl group substituted by 1-3 of substituents optionally selected from a 4 to 7-membered hetero ring containing one phenyl group, a C4-7 cycloalkyl group, a naphthyl group, and a nitrogen atom, (4) a C2-6 alkenyl group substituted by 1-3 of substituents optionally selected from a 4 to 7-membered hetero ring containing one phenyl group, a C4-7 cycloalkyl group, a naphthyl group, and a nitrogen atom, (5) a group represented by the formula R³⁻⁷R³⁻⁸ (wherein R³⁻⁷ and R³⁻⁸ independently represent a 4 to 7-membered hetero ring containing one phenyl group, a C4-7 cycloalkyl group, a naphthyl group, or a nitrogen atom), or (6)a group represented by the formula (C1-6 alkylene)-NR³⁻⁷R³⁻⁸ (wherein R³⁻⁷ and R³⁻⁸ are as defined above, with the proviso that the ring in R³⁻² may be substituted by 1-3 of C1-4 alkyl groups, C1-4 alkoxy groups, halogen atoms, nitro groups, or a trifluoromethyl group) or a salt or a solvate thereof.

Of the compounds represented by the formula (III), examples of more preferable compounds include 2-[5-[2-[N-(diphenylmethyl)carbamoyl]ethyl]naphthalen-1-yloxy] acetic acid or a salt or a solvate thereof.

Moreover, examples of the compound having an agonistic activity to EP3 of the present invention include the compounds described in the pamphlet of WO 97/05091. Of those, examples of preferable compounds include a compound represented by the formula (IV): 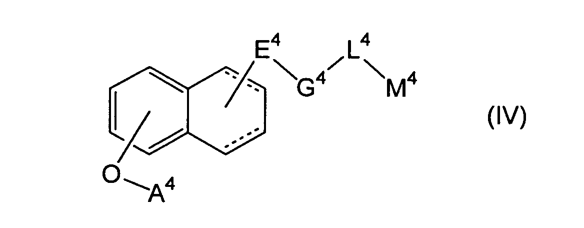 [wherein A⁴ represents a hydrogen atom, a -(C1-4 alkylene) -COOR⁴⁻¹ group (wherein R⁴⁻¹ represents a hydrogen atom or a C1-4 alkyl group), a - (C1-4 alkylene) -CONR⁴⁻²R⁴⁻³ group (wherein R⁴⁻² and R⁴⁻³ each independently represent a hydrogen atom or a C1-4 alkyl group), a - (C1-4 alkylene) -OH group, a - (C1-4 alkylene) -tetrazolyl group, or a -(C1-4 alkylene)-CN group, E⁴ represents a single bond or a C1-6 alkylene group, G⁴ represents -S-, -SO-, -SO₂-, -O-, or a -NR⁴⁻⁴- group (wherein R⁴⁻⁴ represents a hydrogen atom or a C1-4 alkyl group), L⁴ represents a C1-6 alkylene group, a-(CH₂)_{mc}-CH=CH-(CH₂)_{nd}- group (wherein mc is 0 or an integer of 1 to 3, and nd is 0 or an integer of 1 to 3), or a-(CH₂)ₓₐ-CH(OH)-(CH₂)_{ya}- group (wherein xa is an integer of 1 to 3, and ya is 0 or an integer of 1 to 3), M⁴ represents 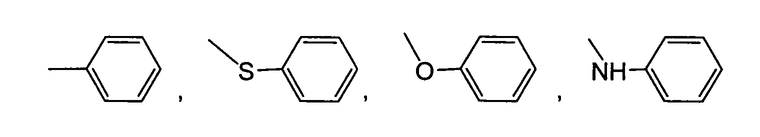 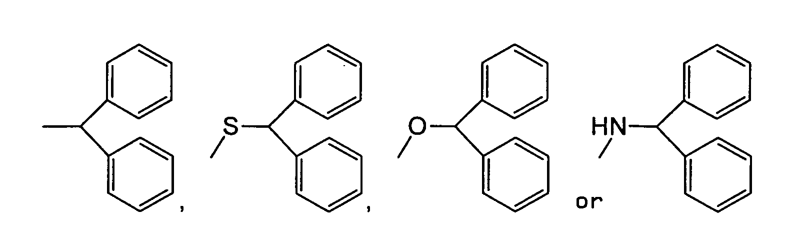 (wherein the respective phenyl groups may be substituted by 1-3 of C1-4 alkyl groups, C1-4 alkoxy groups, halogen atoms, nitro groups, or trifluoromethyl groups), in the following formula: 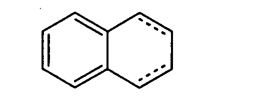 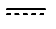 represents a single bond or a double bond, with the proviso that (1) when G⁴ is a -SO- or SO₂- group, M⁴ does not represent 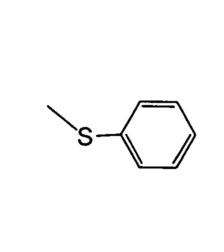 or 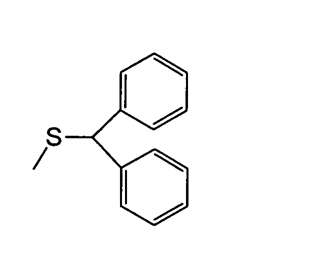 (wherein the respective phenyl groups may be substituted by 1-3 of C1-4 alkyl groups, C1-4 alkoxy groups, halogen atoms, nitro groups, or trifluoromethyl groups), (2) when mc in L⁴ is 0, G⁴ represents a -SO- or SO₂- group, (3) when nd in L⁴ is 0, M⁴ represents 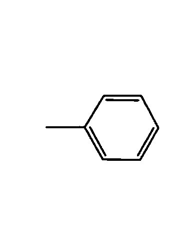 or 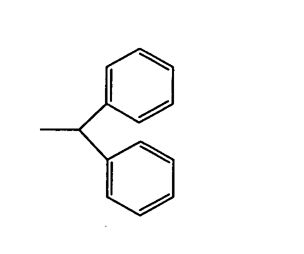 (wherein the respective phenyl groups may be substituted by 1-3 of C1-4 alkyl groups, C1-4 alkoxy groups, halogen atoms, nitro groups, or trifluoromethyl groups), (4) when ya in L⁴ is 0, M⁴ represents 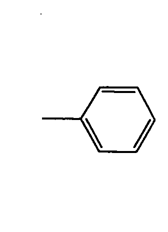 or 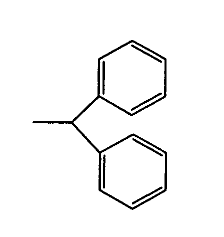 (wherein the respective phenyl groups may be substituted by 1-3 of C1-4 alkyl groups, C1-4 alkoxy groups, halogen atoms, nitro groups, or trifluoromethyl groups), (5) when A⁴ is a hydrogen atom, L⁴ represents a -(CH₂)_{mc}-CH=CH-(CH₂)_{nd}- group (wherein mc and nd are as defined above) or a -(CH2)ₓₐ-CH(OH)-(CH₂)_{ya}- group (wherein xa and ya are as defined above), (6) the tetrazolyl group in A⁴ represents 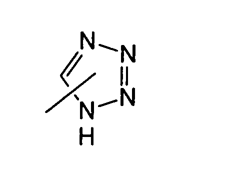 ] or a salt or solvate thereof.

The term "C1-4 alkyl group" represented by R⁴⁻¹, R⁴⁻², R⁴⁻³, and R⁴⁻⁴ in the formula (IV) or "C1-4 alkyl group" as a substituent of a phenyl group in M⁴ means methyl, ethyl, propyl, butyl, and isomer groups thereof, the term "C1-4 alkylene group" in A⁴ means methylene, ethylene, trimethylene, tetramethylene, and isomer groups thereof, the term "C1-6 alkylene group" represented by E⁴ and L⁴ means methylene, ethylene, trimethylene, tetramethylene, pentamethylene, hexamethylene, and isomer groups thereof, the term "C1-4 alkoxy group" in M¹³ means methoxy, ethoxy, propoxy, butoxy, and isomer groups thereof, the term "halogen atom" in M⁴ means a chlorine, a bromine, a fluorine, or a iodine atom, the position to be bound to a side chain represented by -O-A⁴ may be any one of positions 1 to 4, preferably position 1, and the position of a side chain represented by -E⁴-G⁴-L⁴-M⁴ may be any one of positions 5 to 8, preferably position 5 or 6.

Moreover, examples of the compound having an agonistic activity to EP3 of the present invention include the compounds described in the pamphlet of WO 99/25358. Of those, examples of preferable compounds include a compound represented by the formula (V): 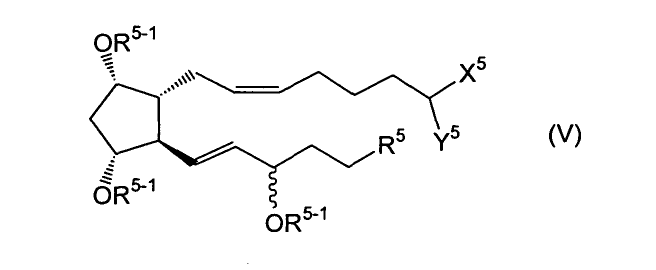 [wherein R⁵ is a substituted heteroaryl group having at least two pendant substituents, and the pendant substituents are selected from the group consisting of a C1-6 alkyl, a halogen atom, trifluoromethyl, COR⁵⁻¹, COCF₃, SO₂NR⁵⁻¹, NO₂, and CN, or R⁵ is a substituted a heteroaryl group having at least one cyano group, R⁵⁻¹ is a hydrogen atom or a C1-6 lower alkyl group, X⁵ is a group selected from the group consisting of -OR⁵⁻¹ and -N (R⁵⁻¹) ₂, and Y⁵ is =O or two hydrogen atoms] or a salt or a solvate thereof.

Moreover, examples of the compoundhaving an agonistic activity to EP3 of the present invention include the compounds described in the publication of JP-A 11-012249. Of those, examples of preferable compounds include a compound represented by the formula (VI) : 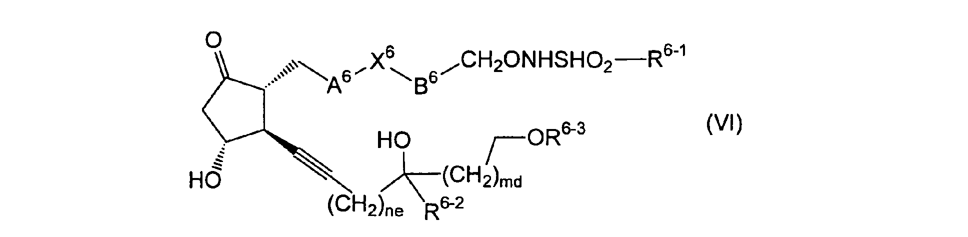 [wherein A⁶ represents a group represented by the following formula: 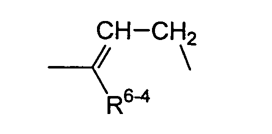 (wherein R⁶⁻⁴ represents a hydrogen atom, a C1-4 alkyl group, or a halogen atom) or a group represented by the following formula: 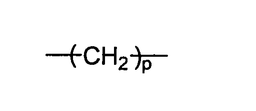 (wherein p is 0 or an integer of 1 to 3), B⁶ represents a group represented by the following formula: 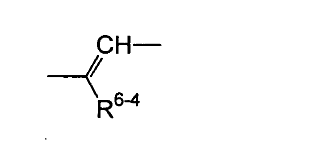 (wherein R⁶⁻⁴ has the same meanings as defined above) or a group represented by the following formula: 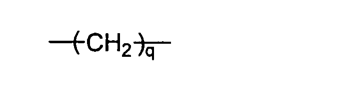 (wherein q is an integer of 1 to 4), X represents a methylene group, an oxygen atom, or a sulfur atom, R⁶⁻¹ represents a C1-4 alkyl group, a phenyl group, or a phenyl group substituted by (a C1-4 alkyl group, a C1-4 alkoxy group, a halogen atom, or a C2-5 alkanoyl group), R⁶⁻² represents a hydrogen atom or a C1-4 alkyl group, R⁶⁻³ represents a C1-4 alkyl group, a phenyl group, or a benzyl group, and ne and md each are 0 or 1] or a salt or a solvate thereof.

Moreover, examples of the compoundhaving an agonistic activity to EP3 of the present invention include the compounds described in the publication of JP-A 10-168056. Of those, examples of preferable compounds include a compound represented by the formula (VII): 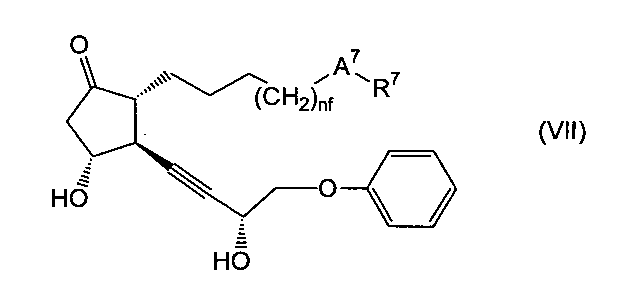 [wherein A⁷ represents an ethylene group, a vinylene group, or an ethynylene group, R⁷ represents a group represented by the following formula: 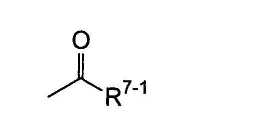 (wherein R⁷⁻¹ represents a C1-4 alkyl group or a C3-8 cycloalkyl group) or a group represented by the following formula: 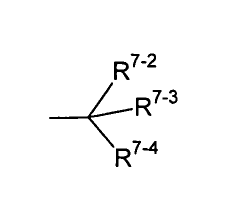 (wherein R⁷⁻² and R⁷⁻³ are the same or different and each represent a hydrogen atom or a C1-4 alkyl group, R⁷⁻⁴ represents a C1-4 alkyl group, a C3-8 cycloalkyl group, a C1-4 alkoxy group, a C3-8 cycloalkoxy group, a hydroxy group, a C1-4 hydroxyalkyl group, a C2-8 acyloxy group, a C1-4 alkylthio group, a C1-4 alkylsulfinyl group, a nitro group, or an acetylamino group), and nf is 0 or 1] or a salt or a solvate thereof.

Moreover, examples of the compoundhaving an agonistic activity to EP3 of the present invention include the compounds described in the publication of JP-A 7-233145. Of those, examples of preferable compounds include a compound represented by the formula (VIII): 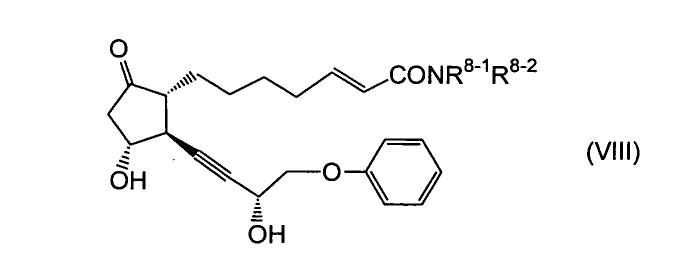 [wherein R⁸⁻¹ and R⁸⁻² are the same or different and each represent a methyl group, a monovalent group of a C7-12 bridged cyclic hydrocarbon, a C1-6 alkylsulfonyl group, or a methoxycarbonylmethyl group substituted by a hydrogen atom, a C1-6 alkyl group, a C3-8 cycloalkyl group, or a C3-8 cycloalkyl group, or R⁸⁻¹ and R⁸⁻² represent a monovalent group of a heterocyclic compound together with an adjacent nitrogen atom] or a salt or a solvate thereof.

Moreover, examples of the compoundhaving an agonistic activity to EP3 of the present invention include the compounds described in the specification of US 4692464. Of those, examples of preferable compounds include a compound represented by the formula (IX): 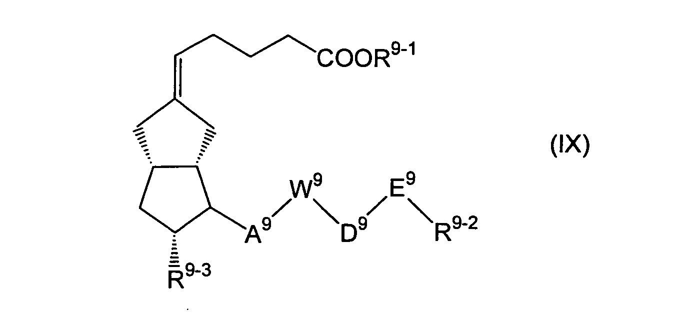 [wherein R⁹⁻¹ represents a hydrogen atom or a C1-4 alkyl group, A⁹ represents a trans -CH=CH-, W⁹ represents a free hydroxymethyl group or a hydroxymethyl group protected by a tetrahydropyranyl group, D⁹ represents a linear or branched alkyl group having 1-5 of carbon atoms, E⁹ represents a -C≡C- bond, R⁹⁻² represents a C1-2 alkyl group, and R⁹⁻³ represents a free hydroxy group or a hydroxy group protected by a tetrahydropyranyl group] or a salt or a solvate thereof.

Of compounds represented by the formula (IX), examples of more preferable compounds include (1S,5S,6R,7R)-5-[7-hydroxy-6-[3(S)-hydroxy-3-methyl-1(E)-octen yl]bicyclo[3.3.0]oct-2-en-3-yl]pentanoic acid (the compound is also referred to as TEI-3356) or a salt or solvate thereof.

Moreover, examples of the compound having an agonistic activity to EP3 of the present invention include the compounds described in the publication of JP-A 51-125255. Of those, examples of preferable compounds include a compound represented by the formula (X) : 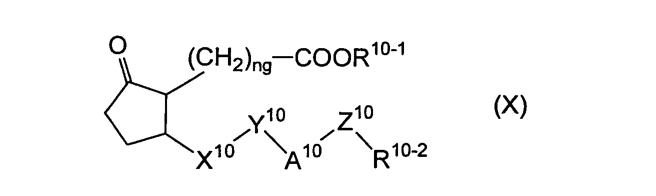 [wherein R¹⁰⁻¹ represents a hydrogen atom or a C1-12 linear or branched alkyl group, R¹⁰⁻² represents an aryl group or a heterocyclic group, and those are substituted by one or more substituents selected from a halogen atom, a C1-4 linear or branched alkyl group, a trihalomethyl group, a C2-4 alkenyl group, a phenyl group, a C1-4 alkoxy group, hydroxyl group, nitro group, cyano group, carboxy group, alkoxycarbonyl group having a C1-4 alkoxy moiety, a hydroxymethylene group, an alkoxymethylene group having a C1-4 alkoxymoiety, a sulfino group, an alkylsulfonyl group having a C1-4 alkoxy moiety, and sulfamoyl, carbamoyl, N-aminocarbamoyl, amidino, amino, and hydroxyimino groups (each nitrogen-containing group above may be substituted by one or more C1-4 alkyl groups), ng is an integer of 5 to 8, (1) A¹⁰ represents a C1-12 linear or branched alkylene group, X¹⁰ represents an ethylene group or trans-vinylene group, Y¹⁰ represents a carbonyl group or -CH (OR¹⁰⁻³) - group (wherein R¹⁰⁻³ represents a hydrogen atom or calboxyl-type acyl group), Z¹⁰ represents a direct bond, an oxygen atom, or asulfur atom, or (2) both A¹⁰ and Z¹⁰ represent direct bonds, X¹⁰ and Y¹⁰ together represent ethylene and carbonyl, trans-vinylene and carbonyl, or ethylene and a -CH (OR¹⁰⁻³)- group (wherein R¹⁰⁻³ has the definition above), respectively] or a salt or a solvate thereof.

Of compounds represented by the formula (X), examples of more preferable compounds include (+/-)-15α-hydroxy-9-oxo-16-phenyoxy-17,18,19,20-tetranorprost-13-trans-enoic acid (the compound is also referred to as M&B 28767) or a salt or solvate thereof.

Moreover, examples of the compoundhaving an agonistic activity to EP3 of the present invention include the compounds described in the publication of JP-A 61-249951. Of those, examples of preferable compounds include a compound represented by the formula (XI) : 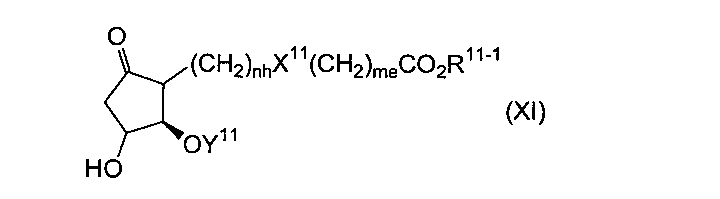 [wherein nh is 1 or 2, me is an integer of 2 to 5, X¹¹ is cis or trans -CH=CH- or -CH₂-CH₂-, or me is an integer of 1 to 4, X¹¹ is -CH=C=CH-, R¹¹⁻¹ is (1) a phenyl group (which is optionally substituted by a C1-4 alkyl group, a C1-4 alkoxy group, a C1-4 alkanoyl group, a methylthio group, a methylsulfinyl group, a methylsulfonyl group, a halogen atom, a -CO₂R¹¹⁻² group (wherein R¹¹⁻² is a hydrogen atom, a C1-4 alkyl group, or a phenyl group), a -NHCOR¹¹⁻² group (wherein R²⁰⁻² has the same definition as above or is a phenyl group optionally substituted by a hydroxy group, CH₃CONH-, or benzoylamino group), a -CONR¹¹⁻³R¹¹⁻⁴ group (wherein R¹¹⁻³ and R¹¹⁻⁴ are the same or different, each is a hydrogen atom or a C1-4 alkyl group), -NHCONH₂, -CH₂CH(CONH₂)NHCOCH₃, or the following formula: 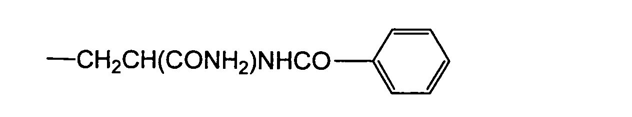 ), or (2) a 2-naphthyl group, Y¹¹ represents a compound represented by the following formula: 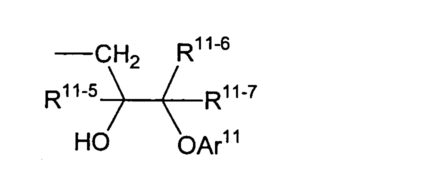 [wherein each of R¹¹⁻⁵, R¹¹⁻⁶, and R¹¹⁻⁷ is a hydrogen atom or a methyl group, at least one group of those is a hydrogen atom, and Ar¹¹ represents a phenyl group (which is optionally substituted by one or two of C1-4 alkyl groups, C1-4 alkoxy groups, C1-4 alkylthio groups, C1-4 alkylsulfinyl groups, C1-4 alkylsulfonyl groups, halogen atoms, or trifluoromethyl groups)] or a salt or a solvate thereof.

Of compounds represented by the formula (XI), examples of more preferable compounds include 4-(benzoylamino)phenyl (-)-[1(R)-[1α(Z),2β(R*),3α]]-7-[3-hydroxy-2-(2-hydroxy-3-pheno xypropoxy)-5-oxocyclopentyl]-4-heptenate (the compound is also referred to as GR63799X) or a salt or a solvate thereof.

Moreover, examples of the compoundhaving an agonistic activity to EP3 of the present invention include the compounds described in the specification of US 4863961. Of those, examples of preferable compounds include a compound represented by the formula (XII): 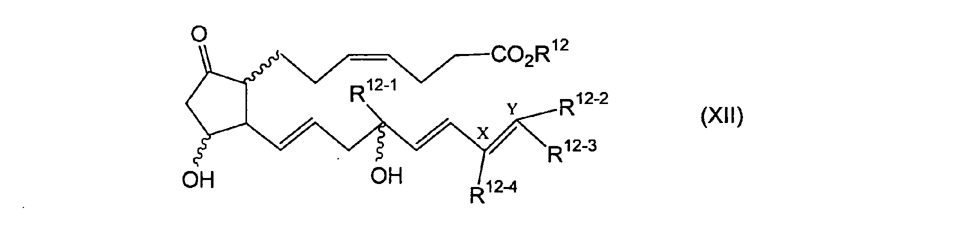 [wherein R¹² represents a hydrogen atom or a C1-4 alkyl group, R¹²⁻¹ represents a hydrogen atom, vinyl, or a C1-4 alkyl group, the wavy line represents R or S stereochemistry, each of R¹²⁻², R¹²⁻³, and R¹²⁻⁴ is a hydrogen atom or a C1-4 alkyl group, R¹²⁻² and R¹²⁻³ form a C4-6 cycloalkenyl group together with a carbon atom Y, or R¹²⁻² and R¹²⁻⁴ form a C4-6 cycloalkenyl group together with carbon atoms X and Y] or a salt or a solvate thereof.

Of compounds represented by the formula (XII), examples of more preferable compounds include methyl-7-(2β-(6-(1-cyclopentyl-yl)-4R-hydroxy-4-methyl-1E,5E-h exadienyl)-3α-hydroxy-5-oxo-1R,1α-cyclopentyl)-4Z-heptenoic acid (the compound is also referred to as SC-46275) or a salt or a solvate thereof.

Moreover, examples of the compoundhaving an agonistic activity to EP3 of the present invention include the compounds described in the specification of US 3985791. Of those, examples of preferable compounds include a compound represented by the formula (XIII): 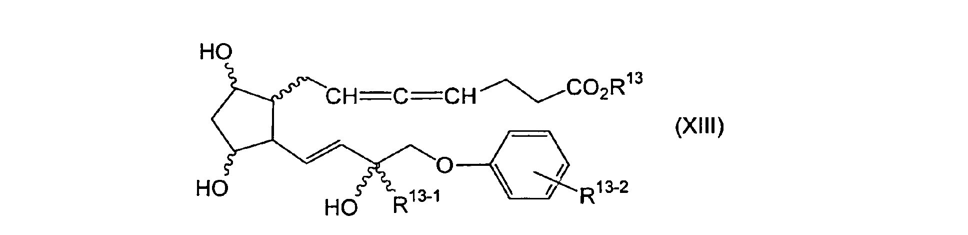 [wherein R¹³ represents a hydrogen atom or a C1-4 alkyl group, R¹³⁻¹ represents a hydrogen atom, a methyl group, or an ethyl group, R¹³⁻² represents a hydrogen atom, an ortho, meta, or para-halogen atom (a fluorine atom, a chlorine atom, or a bromine atom), an ortho, meta, or para-trifluoromethyl group, an ortho, meta, or para-alkyl group, or an ortho, meta, or para-alkoxy group, with the proviso that when R¹³⁻¹ is in the α-configuration, the hydroxyl group bound to the same carbon atom is in the β-configuration, and when R¹³⁻¹ is in the β-configuration, the hydroxyl group bound to the same carbon atom is in the α-configuration] or a salt or a solvate thereof.

Of compounds represented by the formula (XIII), examples of more preferable compounds include methyl 9-oxo-11α,15α-dihydroxy-16-phenoxy-17,18,19,20-tetranorprosta-4,5,13-trans-trienoate (the compound is also referred to as Enprostil) or a salt or a solvate thereof.

Furthermore, of the compounds having an agonistic activity to EP3 of the present invention, examples of more preferable compounds include 16-phenoxy-ω-17, 18, 19, 20-tetranor-PGE2 methylsulfonamide (the compounds is also referred to as Sulprostone) or a salt or a solvate thereof.

Moreover, examples of the compoundhaving an agonistic activity to EP3 of the present invention include the compounds described in the specification of US 3965143. Of those, examples of preferable compounds include a compound represented by the formula (XIV): 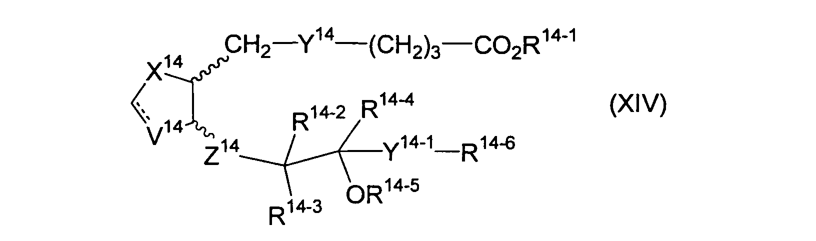 [wherein each of R¹⁴⁻¹, R¹⁴⁻², and R¹⁴⁻³ is a hydrogen atom or C1-7 alkyl group, R¹⁴⁻⁴ is a C1-7 alkyl group, R¹⁴⁻⁵ is a hydrogen atom, C1-7 alkyl group, or C1-7 alkanoyl group, R¹⁴⁻⁶ is a C2-4 alkyl group or C5-7 cycloalkyl group, X¹⁴ is a carbonyl, hydroxymethylene, or an alkanoyloxymethylene group (with the proviso that the alkanoyl moiety includes 1-7 of carbon atoms), V¹⁴ is a methylene, hydroxymethylene, or an alkanoyloxymethylene group (with the proviso that the alkanoyl moiety includes 1-7 of carbon atoms), Y¹⁴ is an ethylene or a vinylene group, Y¹⁴⁻¹ is a vinylene, ethynylene, or the following group: 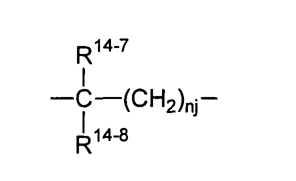 (wherein nj is 0 or 1, each of R¹⁴⁻⁷ and R¹⁴⁻⁸ is a hydrogen atom or a C1-7 alkyl group), and Z¹⁴ is an ethylene, vinylene, or ethynylene group] or a salt or a solvate thereof.

Of compounds represented by the formula (XIV), examples of more preferable compounds include (+/-)-15-deoxy-16-α,β-hydroxy-16-methyl PGE1 methyl ester (the compound is also referred to as Misoprostol) or a salt or a solvate thereof.

Of other compounds having an agonistic activity to EP3 of the present invention, examples of preferable compounds include 15-hydroxy-9-oxo-, (15S)-prostan-1-oic acid (the compound is also referred to as AY23626) or a salt or a solvate thereof.

### [Method of producing compound to be used in the present invention]

A compound of the present invention represented by the formula (I), 11α,15α-dimethoxy-9-oxoprosta-5Z,13E-dienoic acid (which is a more preferable compound), and a salt thereof can be produced in accordance with the method described in the pamphlet of WO98/34916.

A compound of the present invention represented by the formula (II) and a salt thereof can be produced in accordance with the method described in the specification of US 5624959 or US 5723493.

A compound of the present invention represented by the formula (III), 2-[5-[2-[N-(diphenylmethyl)carbamoyl]ethyl]naphthalen-1-yloxy] acetic acid (which is a more preferable compound), and a salt thereof can be produced in accordance with the method described in the specification of US 5753700 or US 6013673.

A compound represented by the formula (IV) and a salt thereof can be produced in accordance with the method described in the pamphlet of WO 97/05091.

A compound represented by the formula (V) and a salt thereof can be produced in accordance with the method described in the pamphlet of WO 99/25358.

A compound represented by the formula (VI) and a salt thereof can be produced in accordance with the method described in the publication of JP-A 11-012249.

A compound represented by the formula (VII) and a salt thereof can be produced in accordance with the method described in the publication of JP-A 10-168056.

A compound represented by the formula (VIII) and a salt thereof can be produced in accordance with the method described in the publication of JP-A 7-233145.

A compound represented by the formula (IX), 5-[(1S,5S,6R,7R)-7-hydroxy-6-[(E)-(S)-4-hydroxy-4-methyl-1-oct enyl]bicyclo[3.3.0]oct-2-en-3-yl]pentanoic acid (which is a more preferable compound), and a salt thereof can be produced in accordance with the method described in the specification of US 4692464.

A compound represented by the formula (X), (+/-)-15α-hydroxy-9-oxo-16-phenoxy-17,18,19,20-tetranorprost-1 3-trans-enoic acid (which is a more preferable compound), and a salt thereof can be produced in accordance with the method described in the publication of JP-A 51-125255.

A compound represented by the formula (XI), [1R-[1α(Z),2β(R*),3α]]-4-(benzoylamino)phenyl-7-[3-hydroxy-2-( 2-hydroxy-3-phenoxypropoxy)-5-oxocyclopentyl]-4-heptenoic acid (which is a more preferable compound), and a salt thereof can be produced in accordance with the method described in the publication of JP-A 61-249951.

A compound represented by the formula (XII), methyl-7-(2β-(6-(1-cyclopentyl-yl)-4R-hydroxy-4-methyl-1E,5E-h exadienyl)-3α-hydroxy-5-oxo-1R,1α-cyclopentyl)-4Z-heptenoic acid (which is a more preferable compound), and a salt thereof can be produced in accordance with the method described in the specification of US 4863961.

A compound represented by the formula (XIII), methyl 9-oxo-11α,15α-dihydroxy-16-phenoxy-17,18,19,20-tetranorprosta-4,5,13-trans-trienoate (which is a more preferable compound), and a salt thereof can be produced in accordance with the method described in the specification of US 3985791 or EP 0139608.

A compound represented by the formula (XIV), (+/-)-15-deoxy-16-α,β-hydroxy-16-methyl PGE1 methyl ester (which is a more preferable compound), and a salt thereof can be produced in accordance with the method described in the specification of US 3965143 or US 4301146.

### [Application to pharmaceuticals]

A compound having an agonistic activity to EP3 is useful in prophylaxis and/or therapy of an allergic disease or an inflammatory disease in a mammal including a human being, particularly a human being. For example, the compound is useful in therapy and/or prophylaxis of an allergic respiratory disease (such as bronchial asthma, pediatric asthma, allergic asthma, atopic asthma, aspirin asthma, or allergic bronchitis), allergic nasal disease (such as allergic rhinitis, vernal catarrh, hay fever, or chronic allergic rhinitis), an allergic skin disease (such as atopic dermatitis), an allergic ocular disease (such as hay fever, seasonal allergic conjunctivitis, or chronic allergic conjunctivitis), chronic bronchitis, reactive airway disease, eosinophilic pneumonia, chronic obstructive pulmonary disease (COPD), sarcoidosis, lung fibrosis, idiopathic interstitial pneumonia, hypersensitivity pneumonitis, rhus dermatitis, contact dermatitis, psoriasis, urticaria, eczema, herpetiform dermatitis, food allergy, inflammatory bowel disease, ulcerative colitis, Crohn's disease, or arthritis. More preferably, the compound is useful in therapy and/or prophylaxis of bronchial asthma.

Of course, the compounds to be used in the present invention include a salt produced in accordance with a known method. The salt is preferably a pharmacologically acceptable salt, and the compounds specified in the present description and claims are confirmed to have low toxicity to the extent of a pharmacologically acceptable compound and to be fully safe for using it as a pharmaceutical product.

The term "pharmacologically acceptable salt" herein includes an alkaline metal salt, alkaline earth metal salt, ammonium salt, amine salt, or the like in the case that a parental compound is an acidic compound, or includes an organic or inorganic acid addition salt or the like in the case that a parental compound is a basic compound.

Moreover, the pharmacologically acceptable salt is preferably water-soluble. Examples of preferable salts include an alkaline metal (such as potassium or sodium) salt, an alkaline earth metal (such as calcium or magnesium) salt, an ammonium salt, pharmacologically acceptable organic amine or amino acid (such as tetramethylammonium, triethylamine, methylamine, dimethylamine, cyclopentylamine, benzylamine, phenetylamine, piperidine, monoethanolamine, diethanolamine, tris(hydroxymethyl)aminomethane, lysine, arginine, or a N-methyl-D-glucamine) salt.

The acid addition salt is preferably water-soluble. Examples of preferable acid addition salts include: inorganic acid salts such as hydrochloride, hydrobromide, hydroiodide, sulfate, phosphate, andnitrate; or organic acid salts such as acetate, lactate, tartrate, benzoate, citrate, methanesulfonate, ethanesulfonate, benzenesulfonate, toluenesulfonate, isethionate, glucuronate, and gluconate.

In addition, the compound to be used in the present invention or a salt thereof may be a solvate.

The solvate is preferably nontoxic and water-soluble. Examples of preferable solvates include solvates such as water and an alcoholic solvent (such as ethanol).

Moreover, the compound to be used in the present invention may be a prodrug produced in accordance with a known method.

A prodrug of the compound to be used in the present invention is a compound to be converted into the compound to be used in the present invention by an in vivo reaction caused by an enzyme or gastric acid. Examples of the prodrug of the compound to be used in the present invention include: when the compound to be used in the present invention has a hydroxyl group, a compound in which the hydroxyl group has been acylated, alkylated, phosphorylated, or borylated (such as a compound in which the hydroxyl group of the compound to be used in the present invention is acetylated, palmitoylated, propanoylated, pivaloylated, succinylated, fumarylated, alanylated, or dimethylaminomethylcarbonylated); or when the compound to be used in the present invention has a carboxy group, a compound in which the carboxy group has been esterified or amidated (such as a compound in which the carboxy group of the compound to be used in the present invention has been ethyl-esterified, phenyl-esterified, carboxymethyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolen-4-yl)methyl-esterified, cyclohexyloxycarbonylethyl-esterified, or methyl-amidated). Those compounds can be produced in accordance with a method known per se. Moreover, the prodrug of the compound to be used in the present invention may be a hydrate or a non-hydrate.

The compound to be used in the present invention or an ester thereof can be converted into a cyclodextrin inclusion compound using α-, β-, or γ-cyclodextrin or a mixture thereof in accordance with the method described in the specification of GB 1351238 or GB 1419221. By converting it into a cyclodextrin inclusion compound, the stability and water solubility increase, so that it is suitable in use as a drug.

The prophylactic and/or therapeutic agent of the present invention is generally administered systemically or topically in an oral or parenteral form.

The dosage depends on the drug tobe used in the present invention, as well as the age, body weight, symptom, therapeutic effect, administration method, and treatment time. In the case of oral administration, the drug is generally administered once or several times a day in an amount of 1 µg to 100 mg per dose per adult. In the case of parenteral administration, the drug is generally administered once or several times a day in an amount of 0.1 ng to 10 mg per dose per adult. The parenteral administration form is preferably intravenous administration, and the drug is intravenously administered sustainably for 1 to 24 hours a day.

Of course, as described above, the dosage varies depending on various conditions, so that a less dosage than the aforementioned one may be sufficient, and a more dosage may be required.

When the prophylactic and/or therapeutic agent of the present invention is administered, it is used as: a solid formulation for internal use, or liquid formulation for internal use for oral administration; and an injection, external preparation, suppository, inhalation, or transnasal agent for parenteral administration.

Examples of the solid formulation for internal use for oral administration include a tablet, a pill, a capsule, powder, and granule. Examples of the capsule include a hard capsule and a soft capsule. Examples of the tablet include a sublingual tablet, an intraoral patch tablet, and an intraoral rapid-disintegrating tablet.

Such a solid formulation for internal use is prepared using one or more active substances without treatment or a mixture of the substance and a vehicle (such as lactose, mannitol, glucose, microcrystalline cellulose, or starch), a binder (such as hydroxypropylcellulose, polyvinylpyrrolidone, or magnesium aluminometasilicate), disintegrator (such as fibrinous calcium glycolate), lubricant (such as magnesium stearate), stabilizer, solubilizing aid (such as glutamic acid or aspartic acid), or the like in accordance with a conventional method. If necessary, it may be coated with a coating agent (such as saccharose, gelatin, hydroxypropylcellulose, or hydroxypropylmethylcellulose phthalate) or coated with two or more layers. In addition, it also encompasses a capsule of an absorbable substance such as gelatin.

The sublingual tablet is produced in accordance with a known method. For example, it is prepared using a mixture of one or more active substances and a vehicle (such as lactose, mannitol, glucose, microcrystalline cellulose, colloidal silica, or starch), a binder (such as hydroxypropylcellulose, polyvinylpyrrolidone, or magnesium aluminometasilicate), disintegrator (such as starch, L-hydroxypropylcellulose, carboxymethylcellulose, croscarmellose sodium, or fibrinous calcium glycolate), lubricant (such as magnesium stearate), a swelling agent (such as hydroxypropylcellulose, hydroxypropylmethylcellulose, carbopol, carboxymethylcellulose, polyvinyl alcohol, xanthan gum, or guar gum), a swelling aid (such as glucose, fructose, mannitol, xylitol, erythritol, maltose, trehalose, phosphate, citrate, silicate, glycine, glutamic acid, or arginine), stabilizer, solubilizing aid (such as polyethylene glycol, propylene glycol, glutamic acid, or aspartic acid), or flavor (such as orange, strawberry, mint, lemon, or vanilla) in accordance with a conventional method. If necessary, it may be coated with a coating agent (such as saccharose, gelatin, hydroxypropylcellulose, or hydroxypropylmethylcellulose phthalate) or coated with two ormore layers. Moreover, if necessary, it may be added with a generally used additive such as a preservative, antioxidant, colorant, or sweetener. The intraoral patch tablet is produced in accordance with a known method. For example, it is prepared using a mixture of one or more active substances and a vehicle (such as lactose, mannitol, glucose, microcrystalline cellulose, colloidal silica, or starch), a binder (such as hydroxypropylcellulose, polyvinylpyrrolidone, or magnesium aluminometasilicate), disintegrator (such as starch, L-hydroxypropylcellulose, carboxymethylcellulose, croscarmellose sodium, or fibrinous calcium glycolate), lubricant (such as magnesium stearate), adhesive (such as hydroxypropylcellulose, hydroxypropylmethylcellulose, carbopol, carboxymethylcellulose, polyvinyl alcohol, xanthan gum, or guar gum), an adhering aid (such as glucose, fructose, mannitol, xylitol, erythritol, maltose, trehalose, phosphate, citrate, silicate, glycine, glutamic acid, or arginine), stabilizer, a solubilizing aid (such as polyethylene glycol, propylene glycol, glutamic acid, or aspartic acid), flavor (such as orange, strawberry, mint, lemon, or vanilla) in accordance with a conventional method. If necessary, it may be coated with a coating agent (such as saccharose, gelatin, hydroxypropylcellulose, or hydroxypropylmethylcellulose phthalate) or coated with two or more layers. Moreover, if necessary, it may be added with a generally used additive such as a preservative, an antioxidant, a colorant, or a sweetener.

The intraoral rapid-disintegrating tablet is produced in accordance with a known method. For example, it is prepared using one or more active substances without treatment or a mixture of: the active substance of bulk powder or granulated bulk powder particles which has been coated with an appropriate coating agent (such as ethylcellulose, hydroxypropylcellulose, hydroxypropylmethylcellulose, or acrylate methacrylate copolymer) or plasticizer (such as polyethylene glycol or triethyl citrate); and a vehicle (such as lactose, mannitol, glucose, microcrystalline cellulose, colloidal silica, or starch), binder (such as hydroxypropylcellulose, polyvinylpyrrolidone, or magnesium aluminometasilicate), disintegrator (such as starch, L-hydroxypropylcellulose, carboxymethylcellulose, croscarmellose sodium, or fibrinous calcium glycolate), lubricant (such as magnesium stearate), a dispersing aid (such as glucose, fructose, mannitol, xylitol, erythritol, maltose, trehalose, phosphate, citrate, silicate, glycine, glutamic acid, orarginine), stabilizer, solubilizing aid (such as polyethylene glycol, propylene glycol, glutamic acid, or aspartic acid), or flavor (such as orange, strawberry, mint, lemon, or vanilla) in accordance with a conventional method. If necessary, it may be coated with a coating agent (such as saccharose, gelatin, hydroxypropylcellulose, or hydroxypropylmethylcellulose phthalate) or coated with two or more layers. Moreover, if necessary, it may be added with a generally used additive such as a preservative, an antioxidant, a colorant, or a sweetener.

Examples of the liquid formulation for internal use for oral administration include a pharmaceutically acceptable solution, suspension, emulsion, syrup, and elixir. In such a liquid formulation, one or more active substances are dissolved, suspended, or emulsified in a generally used diluent (such as purified water, ethanol, or a mixture thereof). Moreover, the liquid formulation may contain a humectant, suspending agent, emulsifier, sweetener, flavor, fragrance, preservative, and buffer.

Examples of the dosage form of the external preparation for parenteral administration include an ointment, gel, cream, poultice, patch, liniment, nebula, inhalant, spray, aerosol, an ophthalmic solution, and a nasal drop. Those contain one or more active substances, and are produced in accordance with a known method or a generally used prescription.

The ointment is produced in accordance with a known or generally usedprescription. For example, it is produced by blending or melting one or more active substances in a base. The base of the ointment is selected from known or generally used ones. For example, there is used a single base or a mixture of two or more of bases selected from a higher fatty acid, higher fatty acid ester (such as adipic acid, myristic acid, palmitic acid, stearic acid, oleic acid, adipate, myristate, palmitate, stearate, or oleate), wax (such as bees wax, whale wax, or ceresin), surfactant (such as polyoxyethylene alkyl ether phosphate), higher alcohol (such as cetanol, stearyl alcohol, or cetostearyl alcohol), silicone oil (such as dimethylpolysiloxane), hydrocarbons (such as hydrophilic petrolatum, white petrolatum, purified petrolatum, and liquid paraffin), glycols (such as ethylene glycol, diethylene glycol, propylene glycol, polyethylene glycol, and macrogol), vegetable oil (such as castor oil, olive oil, sesame oil, or turpentine oil), animal oil (mink oil, egg yolk oil, squalane, orsqualene), water, absorption promoter, and irritation inhibitor. In addition, a humectant, a preservative, stabilizer, antioxidant, flavor, or the like may be contained therein.

The gel is produced in accordance with a known or generally usedprescription. Forexample, itisproducedbyblendingormelting one or more active substances in a base. The base of the gel is selected from known or generally used ones. For example, there is used a single base or a mixture of two or more of bases selected from a lower alcohol (such as ethanol or isopropyl alcohol), gelatinizing agent (such as carboxymethylcellulose, hydroxyethylcellulose,hydroxypropylcellulose, or ethylcellulose), neutralizing agent (such as triethanolamine or diisopropanolamine), surfactant (such as polyethylene glycol monostearate), gums, water, absorption promoter, and irritation inhibitor. In addition, a preservative, an antioxidant, flavor, or the like may be contained therein.

The cream is produced in accordance with a known or generally usedprescription. Forexample, itisproducedbyblendingormelting one or more active substances in a base. The base of the cream is selected from known or generally used ones. For example, there is used a single base or a mixture of two or more of bases selected from a higher fatty acid ester, lower alcohol, hydrocarbons, polyol (such as propylene glycol or 1,3-butylene glycol), higher alcohol (such as 2-hexyldecanol or cetanol), emulsifiers (such as polyoxyethylene alkyl ethers or fatty acid esters), water, absorption promoter, and irritation inhibitor. In addition, a preservative, an antioxidant, flavor, or the like may be contained therein.

The poultice is produced in accordance with a known or generally used prescription. For example, one or more active substances are melted in a base to prepare a kneaded product, and the product is spread and applied onto a support, to thereby produce a poultice. The base of the poultice is selected from known or generally used ones. For example, there is used a single base or a mixture of two or more of bases selected from a thickener (such as polyacrylic acid, polyvinylpyrrolidone, gum Arabic, starch, gelatin, or methylcellulose), humectant (such as urea, glycerin, or propylene glycol), filler (such as kaolin, zinc oxide, talc, calcium, or magnesium), water, dissolution aid, adhesion-imparting agent, and irritation inhibitor. In addition, a preservative, an antioxidant, flavor, or the like may be contained therein.

The patch is produced in accordance with a known or generally used prescription. For example, one or more active substances are melted in a base, and the product is spread and applied onto a support, to thereby produce a patch. The base for the patch is selected from known or generally used ones. For example, there is used a single base or a mixture of two or more of bases selected from a polymer base, fat and oil, higher fatty acid, adhesion-imparting agent, and irritation inhibitor. In addition, a preservative, an antioxidant, flavor, or the like may be contained therein.

The liniment is produced in accordance with a known or generally used prescription. For example, it is produced by dissolving, suspending, or emulsifying one or more active substances in single or two or more substances selected from water, an alcohol (such as ethanol or polyethylene glycol), higher fatty acid, glycerin, soap, emulsifier, and suspending agent. In addition, a preservative, antioxidant, flavor, or the like may be contained therein.

The nebula, inhalant, and spray may contain not only a generally used diluent but also a stabilizer such as sodium hydrogensulfite and an isotonicity-providing buffer such as a isotonicity-adjusting agent including sodium chloride, sodium citrate, or citric acid. Methods of producing a spray are described in detail in the specifications of US 2868691 and US 3095355.

Examples of the injection for parenteral administration include a solid injection, which is dissolved or suspended before use in a solution, suspension, emulsion, and solvent prepared before use. The injection is prepared by dissolving, suspending, or emulsifying one or more active substances in a solvent and used. Examples of the solvent to be used include: distilled water for injection; physiological saline; vegetable oils; alcohols such as propylene glycol, polyethylene glycol, and ethanol; and a combination thereof. The injection may further contain a stabilizer, solubilizing aid (such as glutamic acid, aspartic acid, or polysorbate 80 (registered trademark)), a suspending agent, an emulsifier, a soothing agent, a buffer, a preservative, or the like. Those are produced by sterilization in the last step or by aseptic manipulation. Moreover, an aseptic solid formulation (for example, a freeze-dried product) is produced, and the product may be asepticized before use or dissolved in aseptic distilled water for injection or in another solvent before use.

Examples of the inhalant for parenteral administration include an aerosol, powder for inhalation, or liquid formulation for inhalation. The liquid formulation for inhalation may be dissolved or suspended in water or another appropriate medium before use.

Those inhalants are produced in accordance with a known method.

For example, the liquid formulation for inhalation is optionally prepared from a substance appropriately selected from a preservative (such as benzalkonium chloride or paraben), colorant, buffering agent (such as sodium phosphate or sodium acetate), isotonicity-adjusting agent (such as sodium chloride or concentrated glycerin), thickener (such as a carboxyvinyl polymer), absorption promoter, or the like.

The powder for inhalation is optionally prepared from a substance appropriately selected from a lubricant (such as stearic acid or a salt thereof), a binder (such as starch or dextrin), vehicle (such as lactose or cellulose), a colorant, a preservative (such as benzalkonium chloride or paraben), an absorption promoter, or the like.

In the case of administration of a liquid formulation for inhalation, a spray (atomizer or nebulizer) is generally used, while in the case of administration of a powder for inhalation, administrating equipment for a powder drug is generally used.

Examples of other compositions for parenteral administration include a suppository for intrarectal administration and a pessary for intravaginal administration, which are prescribed in accordance with a conventional method and contain one or more active substances.

A long-lasting preparation may sustainably supply the compound of the present invention to a disease site directly, and examples of the administration form include an implant preparation.

Examples of an in vivo absorbent polymer to be used in a film base of a long-lasting film preparation of a therapeutic agent of the present invention include a fatty acid ester polymer or a copolymer thereof, polyacrylates, polyhydroxybutyrates, polyalkylene oxalates, polyorthoesters, polycarbonates, and polyamino acids. Those may be used singly or as a mixture of two or more of them. Examples of the fatty acid ester polymer or the copolymer thereof include graft, block, alternating, and random copolymers composed of polylactic acid, polyglycol acid, polycitric acid, polymalic acid, poly-ε-caprolactone, polydioxanone, polyphosphazene, or the like, and two or more of them. Those may be used singly or as a mixture of two or more of them. In addition, examples thereof include a polyα-cyanoacrylate, polyβ-hydroxybutyric acid, polytrimethylene oxalate, polyorthoester, polyorthocarbonate, polyethylene carbonate, polyγ-benzyl-L-glutamic acid, and poly L-alanine. There may be used two or more of them, a copolymer with the above-described material, or a mixture of one or two or more of them. Preferable are a polylactic acid, polyglycolic acid, or a lactic acid-glycolic acid copolymer.

Examples of the lactic acid to be used in the lactic acid-glycol acid copolymer include a L-lactic acid and a DL-lactic acid.

The average molecular weight of the above-described in vivo absorbent polymer to be used in the present invention is preferably about 2,000 to about 800,000, more preferably about 5,000 to about 200,000. For example, the weight average molecular weight of a polylactic acid is preferably about 5,000 to about 100,000, more preferably about 6,000 to about 50,000. The polylactic acid can be synthesized in accordance with a producing method known per se.

A lactic acid-glycolic acid copolymer having a composition ratio of lactic acid and glycolic acid of at most about 100/0 to about 0/100 (W/W), more preferably about 90/10 to about 30/70 (W/W) may be used for any purpose. The weight average molecular weight of the lactic acid-glycolic acid copolymer to be used is preferably about 5, 000 to about 100, 000, more preferably about 10, 000 to about 80,000. The lactic acid-glycolic acid copolymer can be synthesized in accordance with a producing method known per se.

The method of producing a film preparation is not particularly limited, but for example, it may be produced: by dissolving the above-described in vivo absorbent polymer and the effective compound of the present invention in an organic solvent, followed by evaporation to dryness, air-drying, or freeze-drying to prepare a film product; by mixing a solution prepared by dissolving the in vivo absorbent polymer in an organic solvent and a solution prepared by dissolving the compound to be used in the present invention in a solvent which is not blended with the organic solvent for emulsification, followed by freeze-drying; or by adding a thickener (such as celluloses or polycarbonates) to a solution prepared by dissolving the in vivo absorbent polymer and the compound to be used in the present invention in an appropriate solvent for gelatinization.

The prophylactic and/or therapeutic agent of the present invention may be used for a disease caused by a cartilage disorder or the like because the effective ingredient (compound) action has sustained-release property, and the sustained-release period is generally one week to three months although the sustained-release period depends on the species of the in vivo absorbent polymer, incorporated amount, or the like. Of those, in particular, in the case of the patients, the affected area is often fixed and plastered. Therefore, it is desired that the healing be sustainably promoted not by frequent administration but by single administration, so that the agent of the present invention is particularly effective.

The dosage of the prophylactic and/or therapeutic agent of the present invention depends on the time for drug sustained-release, animals to be administered, or the like, but has only to be the effective amount of the compound to be used in the present invention. For example, in the case of use in the joint site as a film preparation, about 0.001 mg to 500 mg, preferably about 0.01 mg to 50 mg (which is the dosage per administration) of an effective ingredient may be administered per adult (having a body weight of 50 kg) once a week to three months.

The prophylactic and/or therapeutic agent of the present invention may be used with or prepared with the existing therapeutic agent for an allergic disease or an inflammatory disease for supplementing and/or enhancing its prophylactic and/or therapeutic effect, for improving its dynamics/absorption and decreasing its dosage, and/or for alleviativing side-effects.

Examples thereof include an anti-asthma drug (such as procaterol), inhaled steroid drug (such as beclomethasone, fluticasone, or budesonide), inhaled β2 stimulant (such as fenoterol, salbutamol, formoterol, or salmeterol), methylxanthine asthma drug (such as theophylline), anti-allergy drug (such as suplatast tosilate), histamine H1-antagonist (such as ketotifen fumarate, terfenadine, azelastine hydrochloride, epinastine hydrochloride, oxatomide, mequitazine, emedastine fumarate, astemizole, ebastine, fexofenadine hydrochloride, olopatadine hydrochloride, bepotastine besilate, or cetirizine hydrochloride), antiinflammatory drug (such as diclofenac sodium, ibuprofen, or indomethacin), and anti-choline drug (such as ipratropium bromide, flutropium bromide, oxitropium bromide, or tiotropium bromide). In addition, it may be used with or prepared with a thromboxane antagonist (such as ozagrel hydrochloride, seratrodast, or ramatroban), leukotriene antagonist (such as pranlukast, montelukast, zafirulukast, or zileutin), a mediator release-suppressing drug (such as tranilast, amlexanox, repirinast, ibudilast, tazanolast, or pemirolast), LTD4 antagonist, PAF antagonist, phosphodiesterase inhibitor, β2 agonist, asteroid drug, eosinophil a chemotactic-suppressing drug, DSCG (disodium cromoglycate), macrolide antibiotic (such as erythromycin or roxithromycin), an immune-suppressing agent (such as cyclosporine or FK506), or a hyposensitization therapy agent (specific allergen) .

In use of the prophylactic and/or therapeutic agent of the present invention together with other drugs, those may be administered as a combination agent prepared by incorporating both ingredients in one preparation, or administered as different preparations. In the case of the administration of different preparations, simultaneous administration or differential time administration may be performed. On the other hand, in the differential time administration, other drugs may be administered after administration of the drug of the present invention, or other drugs may be administered before administration of the drug of the present invention. The respective administration methods may be the same or different.

The used amounts of the prophylactic and/or therapeutic agent of the present invention and other drugs are not particularly limited as long as the amounts are used safely. In addition, other drugs for supplementing and/or enhancing the therapeutic effect of the drug of the present invention include known and/or novel compounds.

Moreover, other drugs described herein may have any dosage forms used generally. Examples thereof include solid formulations (such as tablets, tablets, pills, capsules, powders, and granules) and liquid formulations (such as solutions, suspensions, emulsions, syrups, and elixirs).

### Industrial Applicability

A compound having an agonistic activity to EP3 receptor is useful in therapy and/or prophylaxis of an allergic disease or an inflammatory disease in a mammal including a human being, particularly a human being. For example, the compound is useful in therapy and/or prophylaxis of an allergic respiratory disease (such as bronchial asthma, pediatric asthma, allergic asthma, atopic asthma, aspirin asthma, or allergic bronchitis), allergic nasal disease (such as allergic rhinitis, vernal catarrh, hay fever, or chronic allergic rhinitis), an allergic skin disease (such as atopic dermatitis), an allergic ocular disease (such as hay fever, seasonal allergic conjunctivitis, or chronic allergic conjunctivitis), chronic bronchitis, reactive airway disease, eosinophilic pneumonia, chronic obstructive pulmonary disease (COPD), sarcoidosis, lung fibrosis, idiopathic interstitial pneumonia, hypersensitivity pneumonitis, rhus dermatitis, contact dermatitis, psoriasis, urticaria, eczema, herpetiform dermatitis, food allergy, an inflammatory bowel disease, ulcerative colitis, Crohn's disease, or arthritis. More preferably, the compound is useful in therapy and/or prophylaxis of bronchial asthma.

### Brief Description of the Drawings

Fig. 1 shows a therapeutic effect of a compound represented by formula (I-1) that is an agonist to EP3 receptor on asthma by using as an indicator the number of eosinophils in BALF in an OVA-sensitized mouse asthma model.
Fig. 2 shows a therapeutic effect of the compound represented by formula (I-1) that is an agonist to EP3 receptor on asthma by using as an indicator the number of neutrophils in BALF in an OVA-sensitized mouse asthma model.
Fig. 3 shows a therapeutic effect of an agonist to EP3 receptor on asthma by using as an indicator the number of eosinophils in BALF in an OVA-sensitized mouse asthma model.
Fig. 4 shows a therapeutic effect of an agonist to EP3 receptor on asthma by using as an indicator the number of neutrophils in BALF in an OVA-sensitized mouse asthma model.

### Best Mode for carrying out the Invention

Hereinafter, the present invention will be described more detail by way of Example and Formulation Examples relating to the present invention, but the scope of the present invention is not limited thereto.

### Example 1

An asthma reaction was induced using a wild-type mouse (C57/BL6) that had previously been sensitized with an antigen of OVA twice (day 0 and day 12) by inhalation sensitization at intervals of four days a total of three times (day 22, day 26, and day 30). Subsequently, the change in the number of inflammatory cells in BALF that was an indicator of an allergic reaction was examined.

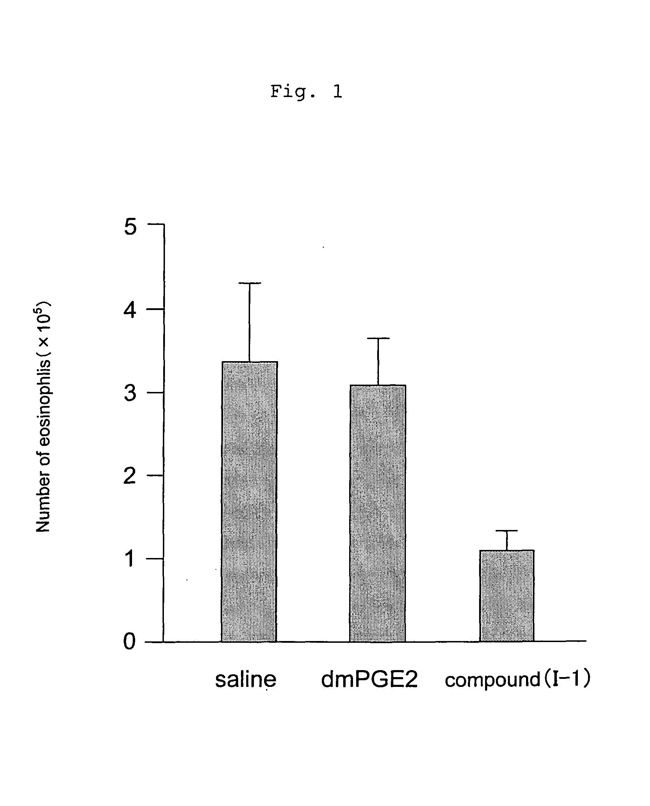

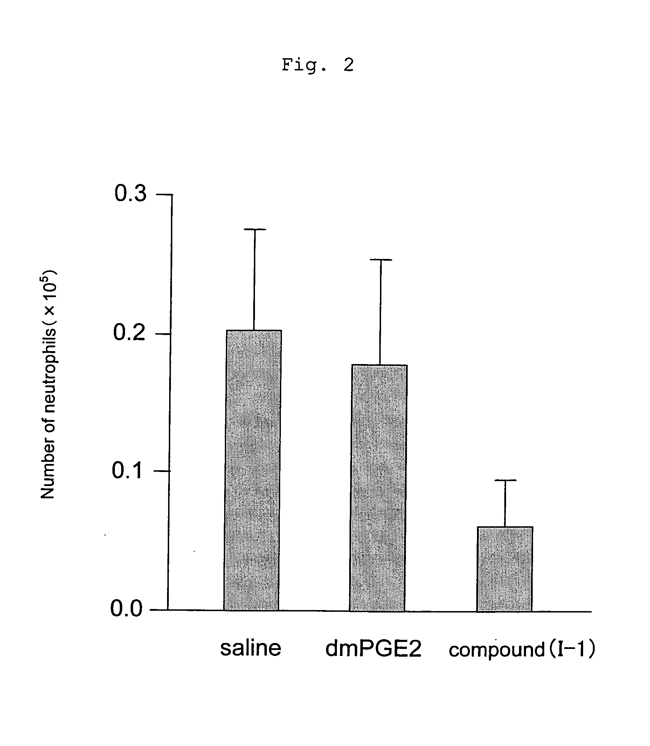

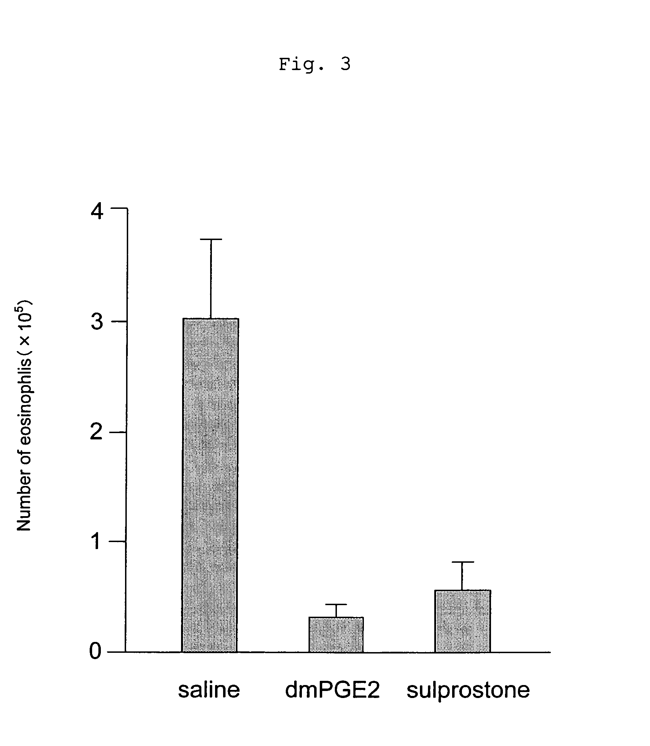

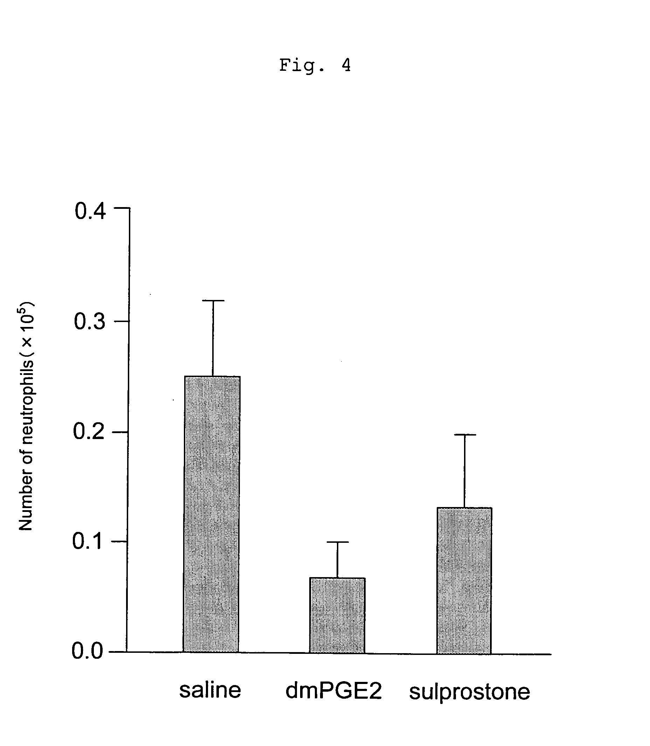

As a result, it was found that the eosinophil and neutrophil numbers in BALF decreased significantly, and an allergic reaction was suppressed when the compound represented by the compound (I-1) : 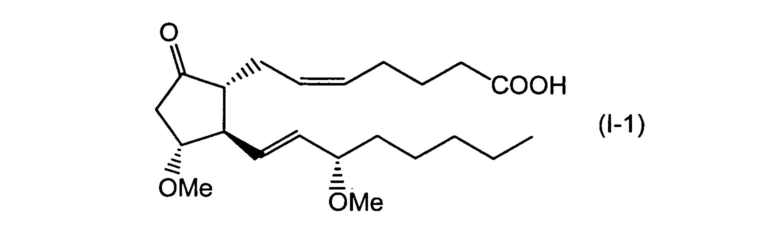 (which is the compound described in Example 1 in the specification of WO 98/34916) having an agonistic activity to EP3 receptor was subcutaneously administered in an amount of 10 µg/kg twice a day for the sensitization period (from day 22 to day 33) (which is shown in Figs. 1 and 2). Meanwhile, when 16,16-dimethyl PGE2 (dmPGE2), which is known as a compoundhaving a non-selective agonistic activity to EP1, EP2, EP3, and EP4 receptors (Br. J. Pharmacol., 122, 217-(1997), Mol. Pharamacol., 59, 1446- (2001)) was subcutaneously administered in an amount of 10 µg/kg twice a day, there was no significant effect (which is shown in Figs. 1 and 2). In addition, when both dmPGE2 and Sulprostone known as a compound having an agonistic activity to an EP1 or EP3 receptor (Br. J. Pharamacol., 122, 217- (1997)) were subcutaneously administered in an amount of 200 µg/kg twice a day, there was a significant effect (which is shown in Figs. 3 and 4). That is, a compound having an agonistic activity to EP3 receptor is effective in therapy of an allergic disease such as asthma, and a selective agonistic compound to EP3 receptor can be expected to induce a more remarkable therapeutic effect.

### Formulation Example 1

The following ingredients were mixed in accordance with a conventional method, and the mixture was then tableted, to thereby yield 100 tablets containing 0.5 mg of an active ingredient per tablet.

| | |
|---|---|
| · 11α,15α-dimethoxy-9-oxoprosta-5Z,13E-dienoic acid | 50 mg |
| · Carboxymethylcellulose calcium | 200 mg |
| · Magnesium stearate | 100 mg |
| · Microcrystalline cellulose | 9.2 g |

### Formulation Example 2

The following ingredients were mixed in accordance with a conventional method, and the solution was sterilized in accordance with a conventional method. Then, a vial was filled with 1 ml of the solution, followed by freeze-drying, to thereby yield 100 vials containing 0.2 mg of an active ingredient per vial.

| | |
|---|---|
| · 11α,15α-dimethoxy-9-oxoprosta-5Z,13E-dienoic acid | 20 mg |
| · Mannitol | 5 g |
| · Distilled water | 100 ml |

## Claims

1. Aprophylactic and/or therapeutic agent for an allergic disease which contains a compound having an agonistic activity to EP3 receptor.

2. The prophylactic and/or therapeutic agent for an allergic disease according to claim 1, in which the allergic disease is an allergic respiratory disease, allergic nasal disease, allergic skin disease, or allergic ocular disease.

3. The prophylactic and/or therapeutic agent for an allergic disease according to claim 2, in which the allergic respiratory disease is bronchial asthma, pediatric asthma, allergic asthma, or atopic asthma, the allergic nasal disease is allergic rhinitis, vernal catarrh, hay fever, or chronic allergic rhinitis, the allergic skin disease is atopic dermatitis, or the allergic ocular disease is seasonal allergic conjunctivitis, hay fever, or chronic allergic conjunctivitis.

4. The prophylactic and/or therapeutic agent for an allergic disease according to claim 2, in which the allergic respiratory disease is bronchial asthma, pediatric asthma, allergic asthma, or atopic asthma.

5. The prophylactic and/or therapeutic agent for an allergic disease according to claim 1, in which the compoundhaving an agonistic activity to EP3 receptor is a compound represented by the formula (I) : 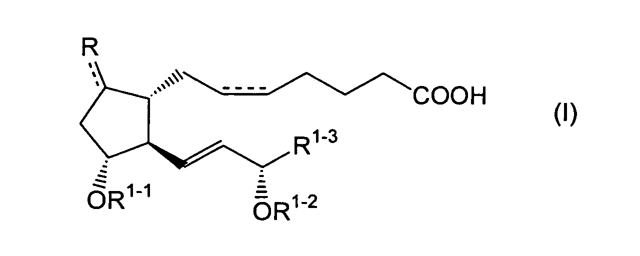 [wherein R represents an oxo group or a halogen atom, R¹⁻¹ and R¹⁻² each independently represent a C1-4 alkyl group, and R¹⁻³ represents a C1-10 alkyl group, a C2-10 alkenylene group, or a C2-10 alkynylene group substituted by a C1-10 alkyl group, a C2-10 alkenylene group, a C2-10 alkynylene group, a phenyl group, a phenoxy group, a C3-7 cycloalkyl group, or a C3-7 cycloalkyloxy group. The phenyl and cycloalkyl groups may be substituted by 1-3 of C1-4 alkyl groups, C1-4 alkoxy groups, halogen atoms, trihalomethyl groups, or nitro groups] or a salt or a solvate thereof.

6. The prophylactic and/or therapeutic agent for an allergic disease according to claim 5, which is 11α, 15α-dimethoxy-9-oxoprosta-5Z, 13E-dienoic acid or a salt or a solvate thereof.

7. The prophylactic and/or therapeutic agent for an allergic disease according to claim 1, in which the compound having an agonistic activity to EP3 receptor is misoprostol or sulprostone;

8. A pharmaceutical composition including the compound having an agonistic activity to EP3 receptor described in claim 1 in combination with one or more drugs selected from anti-asthma drugs, inhaled steroid drugs, inhaled β2 stimulants, methylxanthine asthma drugs, anti-allergy drugs, histamine H1-antagonists, antiinflammatory drugs, anti-choline drugs, thromboxane antagonists, leukotriene antagonists, LTD4 antagonists, PAF antagonists, phosphodiesterase inhibitors, β2 agonist, steroid drugs, mediator release-suppressing drugs, eosinophil chemotactic-suppressing drugs, disodium cromoglycate, macrolide antibiotics, immune-suppressing agent, and hyposensitization therapy agents.

9. A method of preventing and/or treating an allergic disease, which is **characterized by** including administering an effective amount of a compound having an agonistic activity to EP3 receptor to a mammal.

10. Use of a compound having an agonistic activity to EP3 receptor for producing a prophylactic and/or therapeutic agent for an allergic disease.
